# EUROPEAN PATENT APPLICATION

(11) **EP 4 835 068 A1**
(43) Date of publication of application: **04.11.2026**
(21) Application number: 24909466.5
(22) Date of filing: 18.04.2024
(51) Int. Cl.: C12N 9/88, C12N 15/60, C12N 15/70, C12N 1/21, C12P 13/22, C12R 1/19

(54) **ANTHRANILATE SYNTHASE MUTANT AND USE THEREOF**

(30) Priority: 31.12.2023 CN 202311860206
(71) Applicant: Hangzhou Ouhe Biotechnology Co., Ltd., Hangzhou, Zhejiang 310018 (CN)
(72) Inventor: GAO, Qilin, Hangzhou, Zhejiang 310018 (CN); WANG, Qiang, Hangzhou, Zhejiang 310018 (CN); ZHENG, Huabao, Hangzhou, Zhejiang 310018 (CN)
(74) Representative: Metida
(86) International application number: PCT/CN2024/088697
(87) International publication number: WO 2025/138498

(57) **Abstract**

The present disclosure relates to an *Escherichia* coli-derived anthranilate synthase mutant, and a method of preparing the same and use thereof. The mutant is obtained by substituting the last amino acid of a TXLLES domain in an anthranilate synthase derived from *Streptomyces, enterobacteria,* or *Bacillus* with methionine. The mutant has high resistance to feedback inhibition. Compared with the wild-type anthranilate synthase, the inhibition constant *K*ᵢ for tryptophan is increased by more than 400-fold. On this basis, further substituting the serine at position 94 with asparagine can further increase the feedback inhibition resistance to 533-fold relative to the wild type. Compared with the TrpE(S40F) mutant most widely used in current industrial applications, the feedback inhibition resistance is increased by nearly two-fold. In addition, the anthranilate synthase constructed according to the present disclosure has relatively high catalytic activity at pH=6-9, thus having broad applications.

## Description

### TECHNICAL FIELD

The present disclosure relates to the technical field of biotechnology, and particularly to an anthranilate synthase (AS) mutant and use thereof.

### BACKGROUND

Anthranilate synthase is a complex enzyme that catalyzes the reaction of chorismate with an amino group to produce anthranilate, and is one of the key enzymes in the tryptophan biosynthesis pathway. Anthranilate synthase is composed of an *α* subunit and a *β* subunit. The α subunit is a catalytic subunit, and the *β* subunit is an amino donor. The activity of anthranilate synthase is subject to feedback inhibition by tryptophan.

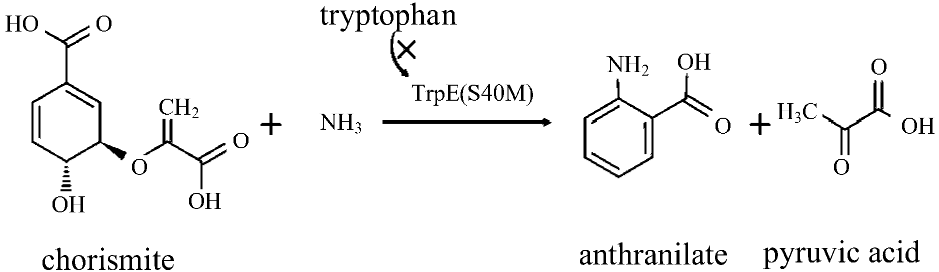

Therefore, changing the structure or expression of anthranilate synthase by metabolic engineering means can increase the content of tryptophan and derivatives thereof in plants, thereby increasing the nutritional value and stress resistance of plants. Research on anthranilate synthase mainly includes biochemical properties, gene structure, regulatory mechanisms, application prospects, and the like. At present, anthranilate synthase mutants reported in the literature and patents for tryptophan production mainly include mutations at several sites, namely substitution of serine at position 40 with phenylalanine (Chen L, Chen M, Ma C, et al. Discovery of feed-forward regulation in L-tryptophan biosynthesis and its use in metabolic engineering of E. coli for efficient tryptophan bioproduction[J]. Metabolic engineering, 2018, 47: 434-444.); substitution of serine at position 40 with arginine (Liu S D, Wu Y N, Wang T M, et al. Maltose utilization as a novel selection strategy for continuous evolution of microbes with enhanced metabolite production[J]. ACS synthetic biology, 2017, 6(12): 2326-2338.); and substitution of alanine at position 63 with valine (Ding D, Bai D, Li J, et al. Analyzing the genetic characteristics of a tryptophan-overproducing Escherichia coli[J]. Bioprocess and Biosystems Engineering, 2021, 44: 1685-1697.). These mutations all have the effect of reducing inhibition of enzyme activity by tryptophan. Among them, the S40F mutant is currently the most widely used and can promote tryptophan production. (Chen L, Chen M, Ma C, et al. Discovery of feed-forward regulation in L-tryptophan biosynthesis and its use in metabolic engineering of E. coli for efficient tryptophan bioproduction[J]. Metabolic engineering, 2018, 47: 434-444. Mindt M, Ferrer L, Bosch D, et al. De novo tryptophanase-based indole production by metabolically engineered Corynebacterium glutamicum[J]. Applied Microbiology and Biotechnology, 2023, 107(5-6): 1621-1634. Kuepper J, Dickler J, Biggel M, et al. Metabolic engineering of Pseudomonas putida KT2440 to produce anthranilate from glucose[J]. Frontiers in Microbiology, 2015, 6: 1310.).

Although these mutants have been reported, no anthranilate synthase mutant capable of completely relieving tryptophan feedback inhibition has yet been found, which may be a key factor limiting current industrial fermentation-based production of L-tryptophan. To solve this problem, there are two approaches. The first approach is to separate the product during tryptophan production so as to reduce the inhibition of anthranilate synthase by tryptophan; however, this method is high in cost and low in efficiency. A more effective method is to mutate potential residues of other anthranilate synthases by biotechnology means to identify mutants that are more resistant to feedback inhibition, thereby increasing the synthesis of anthranilate, a key intermediate in tryptophan production, and achieving the purpose of promoting tryptophan production.

### SUMMARY

In view of the existing problems, the present disclosure provides an anthranilate synthase mutant, and a preparation method and use thereof. Compared with the wild type, the mutant relieves feedback inhibition of enzyme activity by tryptophan. Compared with the wild-type anthranilate synthase, the inhibition constant *K*ᵢ of the mutant for tryptophan is increased by more than 400-fold, and in a preferred embodiment is increased by more than 533-fold.

The present disclosure provides an anthranilate synthase mutant, wherein the last amino acid of a TXLLES domain in an anthranilate synthase derived from *Streptomyces*, *enterobacteria*, or *Bacillus* is substituted with methionine; more specifically, the anthranilate synthase is derived from *Streptomyces coelicolor*, *Escherichia coli*, or *Bacillus subtilis*, wherein X is any amino acid;

more specifically, position 40 of the *Escherichia coli*-derived anthranilate synthase, position 48 of the *Streptomyces coelicolor*-derived anthranilate synthase, or position 50 of the *Bacillus subtilis*-derived anthranilate synthase is substituted with methionine;

further specifically, position 40 of the *Escherichia coli*-derived anthranilate synthase encoded by a nucleotide sequence as set forth in SEQ ID NO: 1 is substituted with methionine; still further, the serine at position 94 is substituted with asparagine.

Preferably, the amino acid sequence of the anthranilate synthase mutant is set forth in SEQ ID NO: 3, SEQ ID NO: 13, SEQ ID NO: 14, or SEQ ID NO: 15.

The present disclosure provides a gene encoding the anthranilate synthase mutant. Preferably, the nucleotide sequence of the gene is set forth in SEQ ID NO: 2. Further, on the basis of the gene of SEQ ID NO: 2, a more preferred gene encoding an anthranilate synthase mutant is disclosed, and the more preferred gene sequence is set forth in SEQ ID NO: 16.

The present disclosure provides a recombinant vector, wherein the recombinant vector comprises the coding gene.

The present disclosure provides a recombinant engineered bacterium comprising the recombinant vector.

The present disclosure further provides a method of preparing an anthranilate synthase mutant, comprising:
S1: inserting the coding gene into a vector plasmid to obtain a recombinant plasmid;
S2: transforming the recombinant plasmid obtained in S1 into competent cells;
S3: subjecting the competent cells of S2 to antibiotic selection to obtain a single colony, and expanding the colony;
S4: subjecting the colony expanded in S3 to fermentation culture;
S5: collecting and disrupting the bacterial cells cultured in S4, and purifying the protein.

Specifically, the vector plasmid in step S1 is pET28a, and the competent cells in step S2 are *Escherichia coli* cells.

The present disclosure further provides use of the anthranilate synthase mutant in preparing tryptophan.

The present disclosure further provides use of the anthranilate synthase mutant in the synthesis of food products, pharmaceuticals, fine chemicals, health products, and cosmetics. Specifically, the use is for relieving feedback inhibition of anthranilate synthase by tryptophan as a final product or intermediate product in the biosynthesis and synthesis of food products, pharmaceuticals, fine chemicals, health products, and cosmetics containing an indole ring.

In summary, compared with the prior art, the present disclosure achieves the following effects: the anthranilate synthase mutant TrpE (S40M) of the present disclosure significantly removes feedback inhibition of enzyme activity by tryptophan; has relatively high catalytic activity at pH 6 to 9; and therefore can be used to promote tryptophan production and reduce production costs.

### BRIEF DESCRIPTION OF THE DRAWINGS

In order to more clearly describe the technical solutions of the embodiments of the present disclosure, the accompanying drawings required for use in the embodiments will be briefly introduced below. It should be understood that the following drawings show only certain embodiments of the present disclosure and therefore should not be regarded as limiting the scope. For a person of ordinary skill in the art, other related drawings may also be obtained based on these drawings without creative effort.
Fig. 1A shows the overlapping conformation of the optimal conformation of molecular docking in Example 1 of the present disclosure and the TrpE-tryptophan co-crystallized molecule derived from *Salmonella typhimurium* (Protein Data Bank (PDB) = 1i1q).
Fig. 1B shows the conformations of three key residues in the docking pocket of *Escherichia coli*-derived anthranilate synthase and tryptophan in Example 1 of the present disclosure.
Fig. 2 shows a map of the recombinant plasmid constructed in Example 1 of the present disclosure.
Fig. 3 shows the protein electrophoresis results of the anthranilate synthase mutant prepared in Example 2 of the present disclosure, wherein the molecular weight of anthranilate synthase is about 58.4 kDa.
Fig. 4 shows determination of the feedback inhibition constant *K*ᵢ of tryptophan against TrpE(S40M), TrpE(S40M/S94N), the wild type, and the previously reported highly efficient mutant TrpE(S40F) constructed in the present disclosure in Example 3 of the present disclosure.
Fig. 5 shows determination of the optimal reaction pH of TrpE(S40M) in Example 4 of the present disclosure.
Fig. 6 is a comparison diagram obtained by ClustalW alignment of the amino acid sequences of anthranilate synthases of *Escherichia coli* and several other representative prokaryotic microorganisms.

### DETAILED DESCRIPTION OF THE EMBODIMENTS

In order to enable those skilled in the art to better understand the solutions of the present disclosure, the technical solutions in the embodiments of the present disclosure will be described clearly and completely below with reference to the accompanying drawings in the embodiments of the present disclosure. Obviously, the described embodiments are merely some, rather than all, embodiments of the present disclosure. All other embodiments obtained by a person of ordinary skill in the art based on the embodiments of the present disclosure without creative effort shall fall within the scope of protection of the present disclosure.

### Example 1: Design and preparation of TrpE mutants

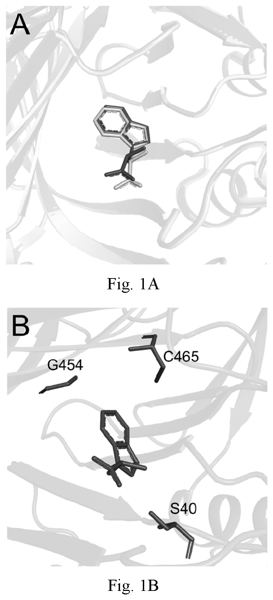

The gene sequence of *Escherichia* coli-derived anthranilate synthase TrpE was obtained from the National Center for Biotechnology Information (NCBI) website and is set forth in SEQ ID NO: 1. Homology modeling of the *Escherichia* coli-derived anthranilate synthase mutant was performed using Swiss-Model (www.swissmodel.expasy.org/); a docking model of tryptophan and *Escherichia* coli-derived anthranilate synthase (TrpE) was constructed using Autodock Vina software; the optimal conformation was selected by comparison with the TrpE-tryptophan co-crystallized molecule derived from *Salmonella typhimurium* (PDB = 1i1q); and mutants were designed by analyzing the docking model of *Escherichia* coli-derived TrpE and tryptophan. As shown in Fig. 1A, the dark portion represents the optimal molecular docking conformation of *Escherichia* coli-derived TrpE with tryptophan, and the light portion represents the TrpE-tryptophan co-crystallized molecular structure derived from *Salmonella typhimurium.* Tryptophan in the two conformations overlaps very well, indicating that the docking conformation has high reliability. Analysis of the tryptophan-binding pocket showed that residues at three sites around tryptophan are closely associated with tryptophan, namely serine at position 40, glycine at position 454, and cysteine at position 465. In order to relieve feedback inhibition of TrpE by tryptophan, it was desired to change these three sites to larger side chains so that the residues and the tryptophan molecule generate steric hindrance, thereby inhibiting binding of tryptophan to the allosteric center. Five mutants were initially designed, namely C465W, G454I, G454Y, G454F, and S40M. As described in the literature, enzyme activities of different mutants were tested in a buffer containing 2 g/L tryptophan, and enzyme activity was defined according to the production rate of the product anthranilate (Lin X, Xu S, Yang Y, et al. Purification and characterization of anthranilate synthase component I (TrpE) from Mycobacterium tuberculosis H37Rv[J]. Protein expression and purification, 2009, 64(1): 8-15.). Through screening, an optimal mutant was obtained and named TrpE(S40M), the gene sequence of which is set forth in SEQ ID NO: 2; the amino acid sequence thereof is set forth in SEQ ID NO: 3.

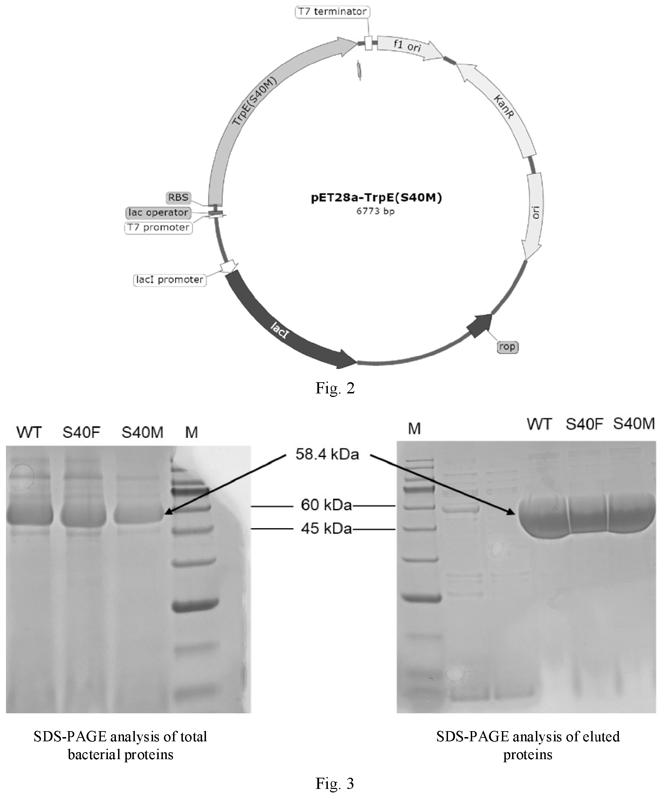

A linearized expression vector was amplified using the pET28a plasmid as a template. The pET28a sequence is set forth in SEQ ID NO: 4, and the primers used are set forth in SEQ ID NO: 5 and SEQ ID NO: 6. The TrpE gene was amplified using the *Escherichia coli* W3110 genome as a template and primers set forth in SEQ ID NO: 7 and SEQ ID NO: 8. The TrpE gene and linearized pET28a were recombined using a ClonExpress II One Step Cloning Kit (Vazyme) to obtain pET28a-TrpE. The sequence of pET28a-TrpE is set forth in SEQ ID NO: 9. The recombinant product was transformed into *Escherichia coli* BL21(DE3) competent cells, plated on a Luria-Bertani (LB) plate containing kanamycin (Kan) resistance, and cultured overnight at 37°C. The correctly identified pET28a-TrpE was used as a template, and pET28a-TrpE(S40M) was constructed using Quick Change technology. The Quick Change primer sequences are set forth in SEQ ID NO: 10 and SEQ ID NO: 11. After the template plasmid was eliminated using Dpn I, the product was transformed into BL21(DE3) competent cells, plated on an LB plate containing Kan resistance, and cultured overnight at 37°C. Each BL21(DE3) engineered bacterium containing pET28a-TrpE (mutant) was identified by sequencing. A map of the pET28a-TrpE(S40M) recombinant plasmid is shown in Fig. 2, and the sequence of the pET28a-TrpE(S40M) recombinant plasmid is set forth in SEQ ID NO: 12.

### Example 2: Expression and purification of TrpE mutants

In order to evaluate the difference between the present mutant and the existing technology, the S40F mutant currently widely used was also constructed in this example with reference to the method in Example 1. From a BL21(DE3) glycerol stock containing a pET28a-TrpE (Wild Type (WT), S40F, or S40M) plasmid, 10 µl of bacterial culture was inoculated into an LB tube containing Kan and cultured overnight at 37°C and 220 rpm. The preculture was inoculated at an inoculation amount of 1% into a fresh 250 ml Terrific Broth (TB) shake flask, and cultured at 37°C and 220 rpm for 3 h until OD600=0.6-0.8. Isopropyl-β-D-thiogalactopyranoside (IPTG) was added to the culture medium to a final concentration of 0.1 mM to induce expression of TrpE and mutant proteins thereof, and the culture was continued at 22°C and 180 rpm for 20 h. A bacterial culture sample was taken for sodium dodecyl sulfate-polyacrylamide gel electrophoresis (SDS-PAGE) to identify whether the protein was overexpressed. Bacterial cells were collected by low-temperature centrifugation at 5000 rpm for 5 min, disrupted, centrifuged, and filtered. Each mutant protein was purified using a Nickel-Nitrilotriacetic acid (Ni-NTA) affinity chromatography column according to a standard procedure. The protein was concentrated and desalted, exchanged into an enzyme storage buffer, and stored at -20°C for later use.

The results are shown in Fig. 3. All three proteins expressed in this example were efficiently expressed in *Escherichia coli*, and expression of wild-type TrpE, the S40M mutant, and the S40F mutant was detected in the eluate. The above results indicate that the preparation method of the anthranilate synthase mutant constructed according to the present disclosure is effective, and that the mutant can be solubly expressed in *Escherichia coli* and has biological activity.

### Example 3: Determination of tryptophan inhibition curves

Experimental steps: a reaction buffer was prepared to contain 100 mM NH₄Cl, 10 mM MgCl₂, 20 mM Tris-HCl, and 0.1 mM ethylenediaminetetraacetic acid (EDTA), and the pH was adjusted to 9.0. 50 ml of the buffer was prepared into an 8 g/L tryptophan buffer, and the pH was adjusted to 9.0. The buffers prepared in the previous two steps were mixed in proportion to obtain tryptophan buffers having different concentrations. In a completely black, opaque microplate, 200 µl of tryptophan buffers having different concentrations were added, and the mutant enzyme solution prepared in Example 2 was added to a final concentration of 0.1 µM, followed by incubation at 30°C for 10 min. Chorismate at a final concentration of 300 µM (large excess) was added to initiate the reaction. A fluorescence microplate reader was used to measure the fluorescence value at 400 nm after excitation at 320 nm, with detection performed once every ten seconds. The microplate reader automatically plotted a curve of fluorescence value over time. The concentration of the reaction product anthranilate and the fluorescence value were in a linear relationship when the anthranilate concentration was within 0-50 µM. The initial slope of the curve plotted by the microplate reader when the tryptophan concentration was 0 was defined as enzyme activity=1.0, and the ratio of the slope under different tryptophan concentrations to the slope without feedback inhibition was defined as relative enzyme activity. The inhibition constant *K*ᵢ value was calculated by fitting using the formula Y= (1-((((Et+X+(*K*ᵢ*(1+(S/*K*ₘ))))-(((Et+X+(*K*ᵢ*(1+(S/*K*ₘ))))²)-4*Et*X)^{0.5}))/(2*Et))), wherein Y is enzyme activity, Et is enzyme concentration, X is inhibitor concentration, S is substrate concentration, and *K*ₘ is the Michaelis constant of the enzyme.

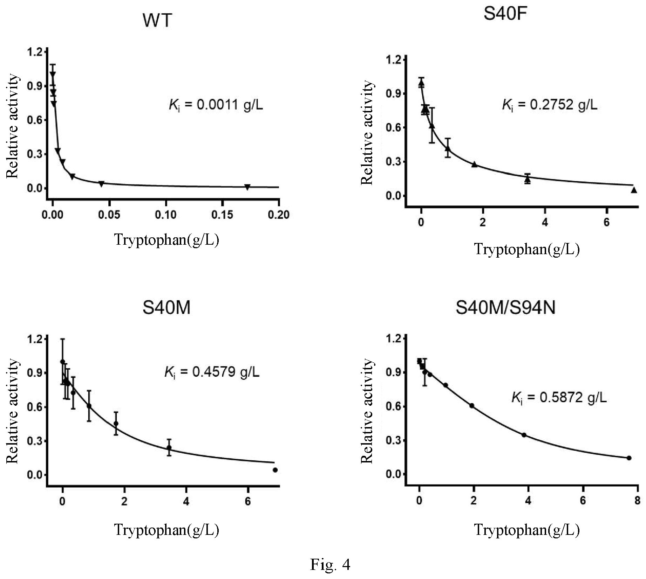

The detection results are shown in Fig. 4. The feedback inhibition constant of tryptophan against S40M, *K*ᵢ=0.4579 g/L, was increased by more than 400-fold compared with that of the wild type (*K*ᵢ=0.0011 g/L). In addition, as determined, S40M constructed according to the present disclosure has stronger feedback inhibition resistance than the currently most widely used TrpE mutant S40F. The *K*ᵢ for inhibition by tryptophan is about 1.7 times that of S40F (*K*ᵢ=0.2752 g/L), indicating broad prospects in industrial tryptophan production.

### Example 4: Effect of pH on TrpE(S40M)

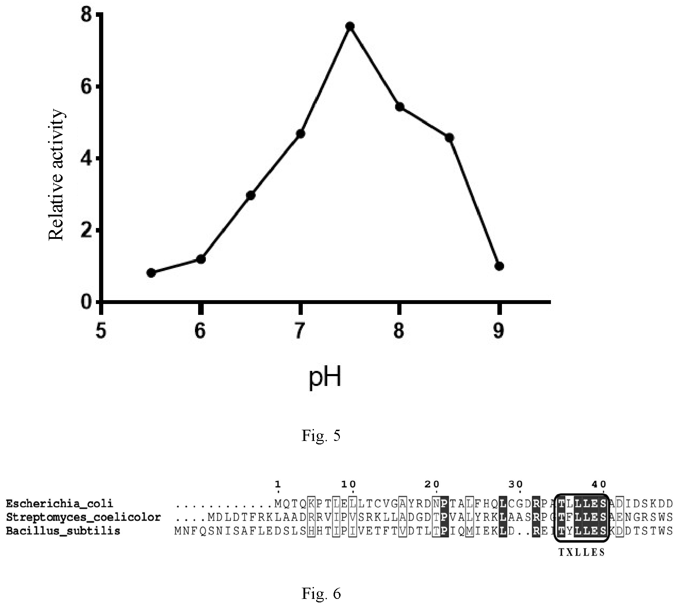

The enzymatic activity of the TrpE(S40M) mutant was measured in buffer systems having different pH values (5.5, 6, 6.5, 7, 7.5, 8, 8.5, and 9), respectively, to determine the optimal pH thereof. In this example, hydrochloric acid and ammonia water were used to adjust the pH value of the reaction buffer, and the reaction and detection method in Example 3 was used to evaluate enzyme activity. The results are shown in Fig. 5. At pH=6-9, TrpE(S40M) had relatively high enzyme activity, and the optimal pH of TrpE(S40M) was 7.5. The above results show that TrpE(S40M) has relatively high enzyme activity under neutral and slightly alkaline conditions.

### Example 5: Anthranilate synthase mutants in other prokaryotic microorganisms

The amino acid sequences of anthranilate synthases of *Escherichia coli* and several other representative prokaryotic microorganisms were aligned using ClustalW. As shown in Fig. 6, the serine at position 40 in the *Escherichia* coli-derived TrpE protein sequence is conserved in *Streptomyces coelicolor and Bacillus subtilis,* and an amino acid sequence domain of TXLLES is present near the tryptophan-binding site, wherein X is any amino acid. To demonstrate that feedback inhibition can be relieved after the last serine in the domain is replaced with methionine, three-dimensional structures of anthranilate synthases derived from *Streptomyces coelicolor and Bacillus subtilis* and TXLLEM were constructed using Swiss-Model. AutodockVina was used to perform molecular docking of the four structures with tryptophan. The docking box size was set to 40×40×40 Å, and the docking box was set near the TXLLES(M) domain; the remaining parameters were default. The docking results were compared with the co-crystallized crystal structure of *Mycobacterium tuberculosis*-derived TrpE with tryptophan to obtain the optimal conformation. Binding energies of the optimal conformations of the respective structures were compared. The results showed that, after the serine at position 48 in the *Streptomyces coelicolor-*derived anthranilate synthase was substituted with methionine, the binding energy with tryptophan increased by 2.0 kJ/mol (-8.2 to -6.2 kJ/mol); after the serine at position 50 in the *Bacillus subtilis-*derived anthranilate synthase was substituted with methionine, the binding energy with tryptophan increased by 2.3 kJ/mol (-8.3 to -6.0 kJ/mol). The above results indicate that the TXLLEM mutation created by the present disclosure also has the effect of reducing tryptophan feedback inhibition in anthranilate synthases of other prokaryotes in addition to *Escherichia coli*. The amino acid sequences of *Streptomyces coelicolor*-derived TrpE(S48M) and *Bacillus subtilis*-derived TrpE(S50M) are set forth in SEQ ID NO: 13 and SEQ ID NO: 14, respectively.

### Example 6: Iterative directed evolution experiment

Using pET28a-TrpE(S40M) as a template, error-prone Polymerase Chain Reaction (PCR) was used to mutate the TrpE gene, and the mutant gene was inserted into pET28a by the method in Example 1 to obtain a mutant library. The mutant library was chemically transformed into TrpD-knockout BL21(DE3) competent cells and cultured overnight at 37°C. 500 clones were randomly selected from the plate, and bacterial culture and protein expression were performed in a 96-well plate with reference to Example 2. After bacterial cells were collected by centrifugation, the cells were resuspended in a buffer containing 2 g/L tryptophan and having pH=9. Chorismate at 5 µM was added using a multi-channel pipette to initiate the reaction. A fluorescence microplate reader was used to continuously measure the fluorescence value at 400 nm after excitation at 320 nm. The clones corresponding to several wells having the highest change in fluorescence value within 10 minutes were sequenced. The results showed that the mutant having the highest activity under inhibition by 2 g/L tryptophan was S40M/S94N (amino acid sequence as set forth in SEQ ID NO: 15), and its DNA sequence is set forth in SEQ ID NO: 16. S40M/S94N was characterized with reference to Examples 2 and 3. As shown in Fig. 4, the feedback inhibition constant of tryptophan against TrpE(S40M/S94N) was 0.5872 g/L, which was increased by 533-fold compared with the wild type, indicating good prospects for industrial application.

## Claims

1. An anthranilate synthase mutant, wherein the last amino acid of a TXLLES domain in an anthranilate synthase derived from *Streptomyces*, *enterobacteria*, or *Bacillus* is substituted with methionine; and more specifically, the anthranilate synthase is derived from *Streptomyces coelicolor*, *Escherichia coli*, or *Bacillus subtilis*, wherein X is any amino acid;
more specifically, position 40 of the *Escherichia coli*-derived anthranilate synthase, position 48 of the *Streptomyces coelicolor*-derived anthranilate synthase, or position 50 of the *Bacillus subtilis*-derived anthranilate synthase is substituted with methionine; and
further specifically, position 40 of the *Escherichia coli*-derived anthranilate synthase encoded by a nucleotide sequence as set forth in SEQ ID NO: 1 is substituted with methionine; and still further, the serine at position 94 is substituted with asparagine.

2. The anthranilate synthase mutant of claim 1, wherein the amino acid sequence of the anthranilate synthase mutant is set forth in SEQ ID NO: 3, SEQ ID NO: 13, SEQ ID NO: 14, or SEQ ID NO: 15.

3. A coding nucleic acid encoding the anthranilate synthase mutant of claim 1 or 2, wherein the coding nucleic acid has a nucleotide sequence set forth in SEQ ID NO: 2 or SEQ ID NO: 16.

4. A recombinant vector comprising the coding nucleic acid of claim 3.

5. A recombinant engineered bacterium comprising the recombinant vector of claim 4.

6. A method of preparing an anthranilate synthase mutant, comprising:
S1: inserting the coding nucleic acid of claim 3 into a vector plasmid to obtain a recombinant plasmid;
S2: transforming the recombinant plasmid obtained in S1 into competent cells;
S3: subjecting the competent cells of S2 to antibiotic selection to obtain a single colony, and expanding the colony;
S4: subjecting the colony expanded in S3 to fermentation culture; and
S5: collecting and disrupting the bacterial cells cultured in S4, and purifying the protein.

7. The preparation method of claim 6, wherein the vector plasmid in step S1 is pET28a.

8. The preparation method of claim 6, wherein the competent cells in step S2 are *Escherichia coli* cells.

9. Use of the anthranilate synthase mutant of claim 1 in preparing tryptophan.

10. Use of the anthranilate synthase mutant of claim 1 in the synthesis of food products, pharmaceuticals, fine chemicals, health products, and cosmetics.
